# FASCICULE DE BREVET EUROPEEN

(11) **EP 0 845 255 B1**
(45) Date de publication et mention de la délivrance du brevet: **13.03.2002**
(21) Numéro de dépôt: 97402866.4
(22) Date de dépôt: 27.11.1997
(51) Int. Cl.: A61K 7/00

(54) **Dispersion aqueuse de vésicules résistantes à la déshydratation**
Wässrige Dispersion von Vesiklen, resistent gegenüber Deshydration
Aquous dispersion of vesicles resistant to dehydration

(30) Priorité: 28.11.1996 FR 9614602
(43) Date de publication de la demande: 03.06.1998
(73) Titulaire: L'OREAL, 75008 Paris (FR)
(72) Inventeur: Simonnet, Jean-Thierry, 75011 Paris (FR)
(74) Mandataire: Stalla-Bourdillon, Bernard

(56) Documents cités:
- WO-A-91/15947
- DE-C- 4 302 132
- GB-A- 2 013 609
- PARFUMS,COSM TIQUES,AROMES, vol. 97, février 1991, PARIS, pages 85-86, XP000201982

## Description

La présente invention concerne une dispersion aqueuse de vésicules résistantes à la déshydratation et de particules de polymère, ainsi qu'une composition cosmétique ou pharmaceutique à application topique à base d'une telle dispersion, ladite composition étant destinée notamment au traitement cosmétique ou dermatologique de la peau.

Par "vésicule", on entend selon l'invention toute structure particulaire comprenant, d'une part, une membrane ou "phase lipidique" constituée par un ou plusieurs feuillets concentriques, ces feuillets comportant une ou plusieurs couches bimoléculaires à base de lipides amphiphiles ioniques ou non ioniques et, d'autre part, une phase aqueuse encapsulée par cette phase lipidique. Au sens de l'invention, les liposomes et les niosomes constituent notamment de telles vésicules.

La structure membranaire des vésicules, telle que celle des liposomes ou des niosomes, en dispersion aqueuse, est particulièrement sensible à tout phénomène de déshydratation, c'est-à-dire tout phénomène entraînant l'élimination, éventuellement totale, du milieu aqueux de la dispersion. Ceci peut se traduire en pratique par une libération indésirable car prématurée d'un produit véhiculé par les vésicules.

Ce problème de résistance à la déshydratation des vésicules en dispersion aqueuse se pose tout particulièrement lors d'une application topique d'une dispersion vésiculaire. En effet, on constate dans ce cas que les vésicules sont non seulement soumises à un cisaillement brutal mais également à une déshydratation d'où, comme cela a été mis en évidence par des études de cryofracture sur peau humaine *in vitro,* un collapsus au moins partiel des vésicules, une fusion avec formation d'un film multilamellaire à la surface d'application et, par voie de conséquence, une libération prématurée du produit véhiculé.

Il est connu de protéger des vésicules des effets de la déshydratation provoquée par la lyophilisation, qui est une technique désormais classique de stockage de vésicules, par addition à la dispersion aqueuse de vésicules, avant lyophilisation, d'une teneur élevée d'un agent cryoprotecteur (environ 15 à 30 % en poids), tel que le sucrose ou le tréhalose. Si ces agents sont reconnus comme étant efficaces, leur présence dans une composition cosmétique ou pharmaceutique à application topique soulève néanmoins certains problèmes du fait de leur teneur élevée dans la composition, ce qui en particulier restreint l'efficacité cosmétique ou pharmaceutique d'une quantité donnée de la composition utilisée mais aussi lui confère un caractère plus ou moins collant tout à fait rédhibitoire pour l'utilisateur lors d'une application topique.

On a donc cherché d'autres moyens permettant d'améliorer la résistance à la déshydratation des vésicules en dispersion aqueuse.

On a maintenant découvert de manière tout à fait surprenante et inattendue qu'il était possible de rendre des vésicules en dispersion aqueuse sensiblement plus résistantes à la déshydratation, notamment lors d'une application topique, en ajoutant un polymère en dispersion soit dans le milieu aqueux de dispersion des vésicules, soit dans la phase aqueuse de gonflement des vésicules dite phase encapsulée ou dans les deux en même temps.

Par l'expression "polymère en dispersion", on entend selon l'invention un polymère sous forme de particules, généralement sphériques, celles-ci étant dispersées dans un milieu aqueux approprié.

La présente invention a donc pour objet une dispersion, dans un milieu aqueux, de vésicules résistantes à la déshydratation constituées d'une phase lipidique et d'une phase aqueuse encapsulée, caractérisée par le fait qu'elle contient en outre, soit dans ledit milieu aqueux, soit dans ladite phase aqueuse encapsulée ou dans les deux en même temps, au moins un polymère en dispersion sous forme de particules, ledit polymère étant choisi dans le groupe constitué par les polymères ayant une température de transition vitreuse Tv, en présence ou non d'un agent plastifiant, inférieure à 70°C à 0 % d'humidité relative ou inférieure à 55°C à 65 % d'humidité relative.

Le polymère utilisé en dispersion selon l'invention doit présenter une température de transition vitreuse Tv répondant aux conditions décrites ci-dessus. On a en effet constaté de manière surprenante et inattendue que seuls les polymères ayant une Tv, en présence ou non d'un agent plastifiant, inférieure à 70°C à 0 % d'humidité relative ou inférieure à 55°C à 65 % d'humidité relative permettaient d'améliorer la résistance à la déshydratation de vésicules en dispersion.

La température de transition vitreuse Tv d'un polymère donné, en présence ou non d'un agent plastifiant, est mesurée de manière connue par analyse thermique différentielle.

Les polymères préférés en dispersion selon l'invention sont ceux dont les particules sont suffisamment stables à l'état dispersé dans le milieu aqueux évitant ainsi la présence d'un agent stabilisant tel qu'un tensioactif, un agent stabilisant hydrophile, un polymère colloïde protecteur ou un mélange de ceux-ci. En effet, l'homme du métier comprendra que la présence d'un agent stabilisant est le plus souvent incompatible avec la nature amphiphile des lipides constituant majoritairement la phase lipidique ou membrane des vésicules dans la dispersion de l'invention. L'absence de tout tensioactif ou autre agent stabilisant est donc un avantage tout à fait non négligeable lors d'une application topique d'une composition cosmétique ou pharmaceutique, du fait de leur caractère irritant.

Compte tenu de ce qui précède, les polymères pouvant être utilisés comme polymère en dispersion selon l'invention peuvent être choisis parmi les polymères synthétiques, les polymères d'origine naturelle, et leurs mélanges.

### 1. Polymères synthétiques

Parmi les polymères synthétiques, on peut citer notamment les polyesters, les polyuréthannes, les polymères à fonctions acides carboxyliques et les polymères vinyliques.

*1.1.* Les polyesters utilisables dans la dispersion selon l'invention peuvent être obtenus, de façon connue, par polycondensation de diacides carboxyliques aliphatiques ou aromatiques avec des diols aliphatiques ou aromatiques ou des polyols. Comme diacides carboxyliques aliphatiques, on peut citer l'acide succinique, l'acide glutarique, l'acide adipique, l'acide pymilique, l'acide subérique ou l'acide sébacique. Comme diacides dicarboxyliques aromatiques, on peut citer l'acide téréphtalique ou l'acide isophtalique, ou bien encore un dérivé tel que l'anhydride phtalique. Comme diols aliphatiques, on peut citer l'éthylène glycol, le propylène glycol, le diéthylène glycol, le néopentyl glycol, le cyclohexane diméthanol, le 4,4'-(1-méthylpropylidène) bisphénol. Comme polyols, on peut citer le glycérol, le pentaérytritol, le sorbitol, le diméthylol propane.

Par polyesters utilisables dans la dispersion selon l'invention, on entend également les polyesteramides, les polyesters à chaîne grasse et les résines époxyesters. Les polyesteramides peuvent être obtenus de manière analogue aux polyesters, mais la polycondensation fait également intervenir une diamine et/ou un aminoalcool. Comme diamine, on peut citer l'éthylène diamine, l'hexaméthylène diamine, la méta- ou para-phénylène diamine. Comme aminoalcool, on peut citer la monoéthanolamine. Les polyesters à chaîne grasse peuvent être obtenus par utilisation de diols à chaîne grasse lors de la polycondensation. Les résines époxyesters peuvent être obtenues par polycondensation d'acides gras avec un condensat aux extrémités α-, γ-diépoxy.

Les polyesters tels que définis ci-dessus peuvent avantageusement comprendre des motifs porteurs de groupements anioniques parmi lesquels on peut citer ceux dérivant de l'acide diméthylol propionique, de l'acide trimellitique ou un dérivé de ceux-ci tel que l'anhydride trimellitique, ou un composé difonctionnel sulfoaryl dicarboxylique substitué sur le noyau aromatique par un groupe -SO₃M dans lequel M représente un atome d'hydrogène ou un ion métallique tel que Na⁺, Li⁺ ou K⁺ comme par exemple le sel de sodium de l'acide sulfo-3 pentanediol, le sel de sodium de l'acide 5-sulfo 1,3-benzène dicarboxylique.

Parmi les polyesters utilisables dans la dispersion selon l'invention, on peut citer plus particulièrement les polyesters et polyesteramides comprenant des motifs dérivant d'un composé difonctionnel sulfoaryl dicarboxylique tel que décrit ci-dessus. De tels polyesters et polyesteramides sont connus et sont notamment décrits dans US-3,779,993, US-4,300,581 et EP-0540374.

Selon un mode de réalisation particulier de la dispersion selon l'invention, le polymère est choisi parmi les polyesters à groupes sulfoniques c'est-à-dire les polyesters comprenant au moins des motifs dérivant d'acide isophtalique, de sels d'acide sulfoaryl dicarboxylique et de diéthyl glycol. Parmi ceux-ci, on peut citer tout particulièrement les polyesters comprenant des motifs d'acide isophtalique, du sel de sodium de l'acide sulfoisophtalique, de diéthylène glycol, et de 1-4 cyclohexane diméthanol, tels que ceux commercialisés sous les dénominations de "AQ29", "AQ38", "AQ48 ultra", "AQ1350", "AQ1045", "AQ1950" et "AQ14000" par la Société EASTMAN CHEMICAL. Ces polyesters peuvent en outre contenir des motifs dérivant d'éthylène glycol, de tri-et tétra-éthylène glycol et de téréphtalate, tels que ceux commercialisés sous les dénominations "Polycare PS 20", "Polycare PS30" et "Polycare PS 32" par la Société RHONE POULENC.

Parmi les polyesters utilisables dans la dispersion selon l'invention, on peut également citer tout particulièrement les polyesters à fonction amine terminale neutralisés par exemple par de l'acide lactique, tels que celui commercialisé sous la dénomination de "Lexamer C120 lactate" par la Société INOLEX ainsi que les copolymères séquencés polyesters du type acide phtalique et sulfophtalique/éthylène glycol/polyméthylsiloxane α,γ-hydroxypropyl tels que celui commercialisé sous la dénomination "PEJ 549" par la Société RHONE POULENC.

*1.2.* Les polyuréthannes utilisables en dispersion selon l'invention peuvent être anioniques, cationiques, non ioniques ou amphotères. Ils peuvent être choisis notamment parmi les polyuréthannes-acryliques, les polyuréthannes-polyvinyipyrrolidones, les polyéther-polyuréthannes, les polyurées, les polyester-polyuréthannes et leurs mélanges.

En particulier, les polyester-polyuréthannes sont notamment ceux décrits dans FR-A-2708615.

*1.3.* Les polymères à fonctions acides carboxyliques utilisables en dispersion selon l'invention sont notamment ceux dont les fonctions acides carboxyliques sont partiellement neutralisées à l'aide d'un agent neutralisant afin de pouvoir assurer un caractère non hydrosoluble suffisant aux particules du polymère.

La neutralisation partielle des polymères à fonctions acides carboxyliques permet par ailleurs d'obtenir avantageusement des polymères en dispersion particulièrement stables en l'absence de stabilisant hydrophile ou de tensioactif ou encore de colloïde protecteur.

L'agent neutralisant est de préférence soit une diamine, soit l'association d'un sel de métal polyvalent et d'une base organique ou minérale. Les diamines sont choisies parmi la lysine, l'arginine ou la cystine. Les sels de métaux polyvalents sont choisis parmi les bromures, chlorures, nitrates, acétates, carbonates et sulfates de calcium, zinc, magnésium, baryum, aluminium et zirconium, et les bases minérales ou organiques sont choisies par exemple parmi la soude, la potasse ou l'ammoniaque ou parmi un aminoalcool tel que l'amino-2 méthyl-2 propanol-1 (AMP), la triéthanolamine, la triisopropanolamine (TIPA), la monoéthanolamine, la diéthanolamine, la tri[(hydroxy-2)propyl-1]amine, l'amino-2 méthyl-2 propanediol-1,3 (AMPD) et l'amino-2 hydroxyméthyl-2 propanediol-1,3, ainsi qu'éventuellement parmi les diamines telles que mentionnées ci-dessus.

Il va de soi que le taux limite supérieur de neutralisation qu'il conviendra de ne pas excéder pour que le polymère à fonctions acides carboxyliques reste insoluble dans l'eau sera fonction de la nature de chaque polymère à fonctions acides carboxyliques et de l'agent neutralisant. De façon générale, lorsque le neutralisant est une diamine, le taux de neutralisation est généralement compris entre 30 et 80% et de préférence entre 40 et 70 si le polymère a moins de 2meq/g de fonctions acides carboxyliques et entre 10 et 50% de préférence entre 10 et 40% si le polymère a plus de 2meq/g de fonctions acides carboxyliques.

Si le neutralisant est une association d'un sel de métal polyvalent et d'une base organique ou minérale, le polymère est d'abord neutralisé par le sel de métal polyvalent à un taux compris entre 4 et 20 %, de préférence entre 4 et 10 %, puis est co-neutralisé avec la base minérale ou organique a un taux de neutralisation totale compris entre 30 et 80 %, de préférence entre 40 et 70 % si le polymère a moins de 2 meq/g de fonctions acides carboxyliques et entre 10 et 50 %, de préférence entre 10 et 40 %, si le polymère a plus de 2 meq/g de fonctions acides carboxyliques.

Ces polymères à fonctions acides carboxyliques, utilisés en dispersion selon l'invention, sont notamment des polymères filmogènes couramment utilisés pour la réalisation de compositions cosmétiques pour la fixation de la chevelure, parmi lesquels on peut citer plus particulièrement :
(i) les copolymères acétate de vinyle/acide crotonique polyoxyéthylénés tels que celui commercialisé sous la dénomination de "Aristoflex A", d'indice d'acide 56, par la Société Hoechst,
(ii) les copolymères acétate de vinyle/acide crotonique tels que celui commercialisé sous la dénomination de "Luviset CA66", d'indice d'acide 65,par la société BASF,
(iii) les terpolymères acétate de vinyle/acide crotonique/ néodécanoate de vinyle, tels que celui commercialisé sous la dénomination de "Résine 28-29-30", d'indice d'acide 65, par la Société National Starch,
(iv) les copolymères N-octylacrylamide/méthacrylate de méthyle/ méthacrylate d'hydroxypropyle/acide acrylique/méthacrylate de tert-butylaminoéthyle, tels que celui commercialisé sous la dénomination de "Amphomer", d'indice d'acide 137, par la Société National Starch,
(v) les copolymères alternés méthylvinyléther/anhydride maléique monoestérifiés par le butanol, tels que celui commercialisé sous la dénomination de "Gantrez ES 425", d'indice d'acide 260, par la Société GAF,
(vi) les terpolymères acide acrylique/acrylate d'éthyle/N-tert-butylacrylamide tels que celui commercialisé sous la dénomination de "Ultrahold 8", d'indice d'acide 62, par la Société BASF, et
(vii) les polymères répondant a la formule générale suivante : 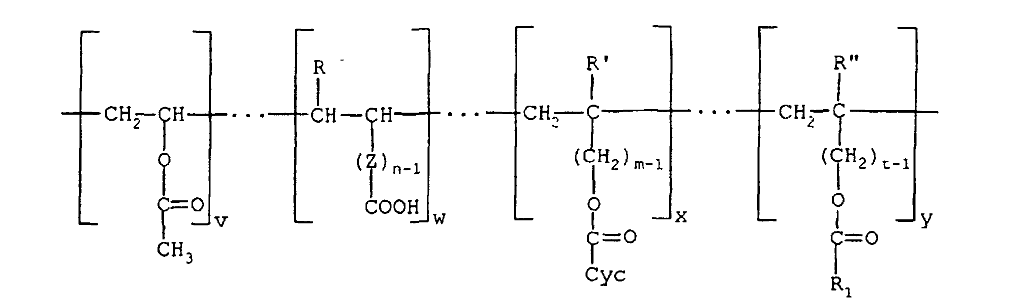
dans laquelle :
R, R' et R", identiques ou différents, représentent un atome d'hydrogène ou un radical méthyle,
m, n et t sont 1 ou 2,
R₁ représente un radical alkyle, linéaire ou ramifié, saturé ou insaturé ayant de 2 à 21 atomes de carbone,
Z représente un radical divalent pris dans le groupe constitué par : -CH₂-, -CH₂-O-CH₂- et -CH₂-O-(CH₂)₂-,
Cyc représente un radical choisi parmi :
   (a) un radical de formule : 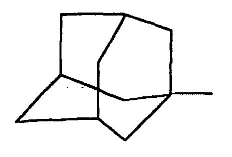
   (b) un radical de formule : 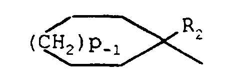
   dans laquelle :
   R₂ représente un atome d'hydrogène ou un radical méthyle,
   et p est 1 ou 2,
   (c) un radical de formule : 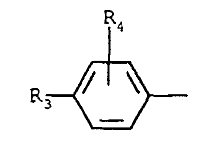
dans laquelle :
R₃ représente un atome d'hydrogène, un radical méthyle, éthyle, tert-butyle, éthoxy, butoxy ou dodécyloxy et R₄ représente un atome d'hydrogène, un radical alkyle de 1 à 4 atomes de carbone ou un radical alkoxy de 1 à 4 atomes de carbone,
et (d) un radical de formule : 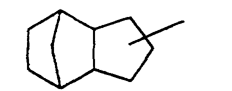
v représente de 10 à 91% et de préférence de 36 à 84% en poids,
w représente de 3 à 20% et de préférence de 6 à 12% en poids,
x représente de 4 à 60% et de préférence de 6 à 40% en poids,
et y représente de 0 à 40% et de préférence de 4 à 30% en poids,
v + w + x + y étant égal à 100%.

Parmi ces polymères, on peut citer notamment le copolymère acétate de vinyle/tert-butyl-4 benzoate de vinyle/acide crotonique (65/25/10) neutralisé à 50 % par de la lysine et le copolymère acétate de vinyle/acide crotonique/tert-butyl-4 benzoate de vinyle (65/10/25) neutralisé à 60 % par de la lysine.

*1.4.* Les polymères vinyliques utilisables en dispersion selon l'invention peuvent résulter de l'homopolymérisation ou de la copolymérisation de monomères choisis parmi les esters vinyliques, le styrène ou le butadiène. Comme exemple d'esters vinyliques, on peut citer l'acétate de vinyle, le néodécanoate de vinyle, le pivalate de vinyle, le benzoate de vinyle et le tert-butyl-4 benzoate de vinyle.

### 2. Polymères naturels

Parmi les polymères d'origine naturelle utilisables en dispersion selon l'invention, on peut citer la résine shellac, la gomme de sandarac, les dammars, les élémies, les copals, les dérivés cellulosiques, et leurs mélanges.

En particulier, les dérivés cellulosiques peuvent être choisis parmi les éthers de cellulose, les esters de cellulose non ioniques et les esters de cellulose anioniques à fonctions acides carboxyliques, lesdites fonctions acides carboxyliques étant neutralisées à un taux de neutralisation compris 10 et 80 % à l'aide d'un agent neutralisant tel que décrit ci-dessus pour les polymères synthétiques à fonctions acides carboxyliques.

Parmi les éthers de cellulose, insolubles dans l'eau, on peut citer notamment les éthylcelluloses, et en particulier celles commercialisées sous les dénominations de "Ethocel" par la Société DOW CHEMICAL.

Parmi les esters de cellulose non ioniques, insolubles dans l'eau, on peut citer notamment les acétates de cellulose, les propionates de cellulose, les butyrates de cellulose, les acéto-propionates de cellulose et les acéto-butyrates de cellulose.

Enfin, parmi les esters de cellulose anioniques à fonctions acides carboxyliques insolubles dans l'eau, on peut citer notamment l'acétophtalate de cellulose, le succinate d'acétate de cellulose, le succinate de propionate de cellulose, le succinate de butyrate de cellulose, le succinate d'acéto-propionate de cellulose, le succinate d'acéto-butyrate de cellulose, le trimellilate d'acétate de cellulose, le trimellilate de butyrate de cellulose, le trimellilate de propionate de cellulose, le trimellilate d'acéto-propionate de cellulose et le trimellilate d'acéto-butyrate de cellulose.

Le polymère utilisable dans la dispersion selon l'invention peut comprendre de façon connue un agent plastifiant, afin notamment de contrôler la température de transition vitreuse Tv du polymère qui doit répondre aux conditions susmentionnées. La quantité d'agent plastifiant est choisie par l'homme du métier sur la base de ses connaissances générales, de manière à obtenir un système polymérique approprié.

Parmi les agents plastifiants pouvant être utilisés, on peut citer :
- les "Carbitols" de la Société UNION CARBIDE à savoir le "Carbitol" ou diéthylène glycol éthyléther, le "méthyl Carbitol" ou diéthylène glycol méthyléther, le "butyl Carbitol" ou diéthylène glycol butyléther ou encore l'"hexyl Carbitol" ou diéthylène glycol hexyléther,
   - les "Cellosolves" de la Société UNION CARBIDE a savoir le "Cellosolve" ou éthylène glycol éthyléther, le "butyl Cellosolve" ou éthylène glycol butyléther, l'"hexyl Cellosolve" ou éthylène glycol hexyléther,
   - les dérivés de propylène glycol et en particulier le propylène glycol phényléther, le propylène glycol diacétate, le dipropylène glycol butyléther, le tripropylène glycol butyléther, ainsi que les "Dowanols" de la Société DOW CHEMICAL à savoir le "Dowanol PM" ou propylène glycol méthyléther, le "Dowanol DPM" ou dipropylène glycol méthyléther et le "Dowanol TPM" ou tripropylène glycol méthyléther.

On peut encore citer :
- le diéthylène glycol méthyléther ou "Dowanol DM" de la Société DOW CHEMICAL,
- l'huile de ricin oxyéthylénée à 40 moles d'oxyde éthylène telle que celle vendue par la Société RHONE POULENC sous la dénomination de "Mulgofen EL-719",
- l'alcool benzylique,
- le citrate de triéthyle vendu par la Société PFIZER sous la dénomination de "Citroflex-2",
- le 1,3-butylène glycol,
- les phtalates et adipates de diéthyle, de dibutyle et de diisopropyle,
- les tartrates de diéthyle et de dibutyle,
- les phosphates de diéthyle, de dibutyle et de diéthyl-2 hexyle, et
- les esters de glycérol tels que le diacétate de glycérol (diacétine) et le triacétate de glycérol (triacétine).

On utilise de préférence un agent plastifiant choisi dans le groupe constitué par le dipropylène glycol méthyléther, le tripropylène glycol méthyléther, l'adipate de diéthyle, l'adipate de diisopropyle, et le triacétate de glycérol.

Le polymère en dispersion, c'est-à-dire la dispersion de particules de polymère dans un milieu aqueux approprié, est préparé soit par polymérisation du ou des monomères correspondants dans un milieu aqueux approprié (on parle alors de latex ou de latex synthétique), soit à partir d'un polymère dans un milieu aqueux approprié (on parle alors de pseudolatex).

Selon un mode de réalisation préféré de la présente invention, le polymère en dispersion est préparé par dispersion d'un polymère dans un milieu aqueux approprié (pseudolatex). Ce procédé de préparation consiste à dissoudre le polymère, insoluble dans l'eau, dans un solvant organique, soluble ou partiellement soluble dans l'eau, à disperser sous agitation la solution ainsi obtenue dans le milieu aqueux approprié et à procéder ensuite à l'élimination du solvant organique par évaporation sous vide. Le polymère insoluble dans l'eau peut également être dispersé dans l'eau à une température supérieure à sa température de transition vitreuse Tv sans adjonction d'un solvant particulier.

Les particules du polymère en dispersion ainsi obtenu ont de préférence une taille moyenne comprise entre 5 et 2000 nm et de préférence encore comprise entre 10 et 500 nm.

Le milieu aqueux approprié pour la préparation du polymère en dispersion peut être de l'eau ainsi que toute autre solution aqueuse.

La dispersion aqueuse de vésicules objet de l'invention peut être préparée selon deux variantes, à savoir, soit en mélangeant simplement un polymère en dispersion à une dispersion aqueuse de vésicules déjà formée, soit en mélangeant une phase lipidique à un polymère en dispersion dont le milieu aqueux provoque la formation de la dispersion de vésicules. On comprendra que l'on peut ainsi obtenir, selon la première variante, une dispersion de vésicules dont le milieu aqueux extérieur de dispersion contient seul des particules de polymère en dispersion et, selon la deuxième variante, une dispersion dans un milieu aqueux de vésicules, dont le milieu aqueux de dispersion ainsi que la phase aqueuse encapsulée des vésicules contiennent des particules de polymère en dispersion.

Quelle que soit la variante utilisée pour la formation de la dispersion objet de l'invention, on utilise comme phase lipidique des vésicules les substances généralement employées pour la préparation des liposomes et niosomes telles que décrites par exemple dans l'article de BANGHAM, STANDISH & WATKINS, J.Mol.Biol. 13, 238 (1965) et dans FR-A-2315991, FR-A-2694884 ou encore FR-A-2714596.

Ainsi, outre les composés lipidiques amphiphiles ioniques ou non ioniques connus, en mélange ou non, la phase lipidique des vésicules peut également comprendre, de façon connue, au moins un additif qui a pour fonction principale de diminuer la perméabilité des vésicules, de prévenir leur fusion ainsi que d'augmenter le taux d'encapsulation.

Cet additif est en général choisi de préférence dans le groupe constitué par les stérols (phytostérol, cholestérol, phytostérol polyoxyéthyléné), les monoalcools, diols et triols à longue chaîne (phytanetriol).

La phase lipidique peut également contenir, de façon connue, au moins un additif qui a pour fonction principale de prévenir la floculation des vésicules obtenues. Cet additif est en général choisi de préférence dans le groupe constitué par les amines à longue chaîne et leurs dérivés ammonium quaternaire, les esters phosphoriques d'alcool gras et leurs sels alcalins (sodium, potassium) tels que le dicétylphosphate, le dicétylphosphate de sodium, les alkylsulfates (cétylsulfate de sodium), les sels alcalins du cholestérol sulfate ou du cholestérol phosphate, le sel de sodium de l'acide phosphatidique, les lipoaminoacides et leurs sels tels que les acylglutamates de sodium (tels que les sels monosodique et disodique de l'acide N-stéaroylglutamique commercialisés sous les dénominations respectives de "Acylglutamate HS 11" et "Acylglutamate HS 21" par la Société AJINOMOTO).

A partir de la phase lipidique décrite ci-dessus, on prépare les vésicules de la dispersion selon l'invention par tout procédé connu et plus particulièrement par le procédé dit "par cofusion des lipides", ce qui permet de les préparer de façon simple à l'échelle industrielle. Ce procédé consiste essentiellement, dans un premier temps, à mélanger les différents lipides et additifs éventuels de la phase lipidique, par fusion et sous agitation et, dans un deuxième temps, à mélanger la phase lipidique obtenue, à chaud et sous agitation rapide, à un milieu aqueux approprié constituant la phase aqueuse à encapsuler, en maintenant l'agitation pendant un temps suffisant. Selon la première variante du procédé, le milieu aqueux peut être de l'eau ou toute autre solution aqueuse et, selon la deuxième variante, le milieux aqueux est celui du polymère en dispersion utilisé tel que décrit précédemment.

La taille des vésicules en dispersion ainsi obtenues peut éventuellement être homogénéisée, par exemple en faisant passer la dispersion vésiculaire dans un homogénéisateur haute-pression, à température ambiante, ou dans un dispositif à ultrasons.

De préférence, la taille moyenne des vésicules ainsi obtenues en dispersion est comprise entre 10 et 5000 nm, et de préférence entre 50 et 1000 nm.

La concentration du polymère dans la dispersion selon l'invention est exprimée en pourcentage en poids de polymère, éventuellement plastifié, par rapport au poids total de la dispersion selon l'invention.

De préférence, cette concentration en polymère est comprise entre 0,01 et 30 % et de préférence entre 0,01 et 5 %.

La concentration des vésicules ou "concentration vésiculaire" dans la dispersion selon l'invention est exprimée en pourcentage en poids de phase lipidique, telle que définie ci-dessus, par rapport au poids total de la dispersion selon l'invention.

De préférence, cette concentration vésiculaire est comprise entre 0,1 et 30 % et encore de préférence entre 0,5 et 10 %.

Selon un mode de réalisation préféré de la présente invention, le rapport pondéral du polymère, sous forme de particules en dispersion, à la phase lipidique est compris entre 1:500 et 20:1, et de préférence entre 1:100 et 1:1.

La résistance à la déshydratation des vésicules de dispersions selon l'invention a été testée vis-à-vis d'une situation extrême de déshydratation des vésicules, à savoir celle engendrée par une opération de lyophilisation.

Ce test de lyophilisation a été effectué dans les conditions suivantes : on a utilisé une phase lipidique de composition stéarate de polyéthylène glycol 400/cholestérol/"Acylglutamate HS 21" (sel disodique de l'acide N-stéaroylglutamique, commercialisé par la Société AJINOMOTO) selon les proportions 45/45/10. La concentration vésiculaire adoptée a été de 3 % en poids de phase lipidique par rapport au poids total de la dispersion finale. La concentration de polymère utilisée a été de 0,3 % en poids de polymère par rapport au poids de la dispersion finale. Les dispersions ainsi obtenues ont ensuite été soumises à une opération classique de lyophilisation : chacune d'entre elles a d'abord été congelée sur les parois d'un ballon rond en rotation dans de l'azote liquide, la dispersion congelée ayant ensuite été placée dans un lyophilisateur pour obtenir ainsi, après un temps suffisant de sublimation du milieu aqueux de dispersion, un produit pâteux de couleur blanche, que l'on peut conserver en tant que lyophilisat. Par réhydratation du lyophilisat avec le même milieu aqueux de dispersion utilisé au préalable pour la préparation de la dispersion, on a obtenu à nouveau une dispersion vésiculaire.

Pour chacune des dispersions ainsi formées, le test de lyophilisation a tout d'abord consisté à vérifier qu'il n'y avait pas recristallisation de certains lipides de la phase lipidique des vésicules (en particulier du cholestérol) et que la dispersion vésiculaire après réhydratation était fine et homogène, d'après un contrôle visuel.

Lorsque ces premières conditions étaient remplies, on a ensuite effectué une mesure granulométrique des vésicules dont le résultat a été comparé à celui d'une mesure effectuée avant l'opération de lyophilisation. On a estimé que l'on obtenait un résultat positif si la taille moyenne des vésicules après lyophilisation n'était pas plus du double de celle avant lyophilisation.

On a ainsi testé dans les conditions décrites ci-dessus les dispersions selon l'invention comprenant chacune un des polymères suivants :
- les polyesters à groupes sulfoniques "AQ29" et "AQ38" commercialisés par la SOCIETE EASTMAN CHEMICAL et les "Polycare PS 20" et "Polycare PS 32" commercialisés par la Société RHONE POULENC;
- le polyester à fonctions amines terminales neutralisé "Lexamer C120 lactate" commercialisé par la Société INOLEX;
- le polyester "PEJ549" commercialisé par la Société RHONE POULENC;
- le copolymère acétate de vinyle/butyl-benzoate de vinyle/acide crotonique selon les proportions (65/25/10) neutralisé à 50 % par de la lysine et comprenant du tripropylène glycol méthyléther à raison de 10 % en poids comme agent plastifiant.

Ces polymères ont chacun été utilisés sous forme d'une dispersion ajoutée d'une part à une dispersion de vésicules déjà formée (première variante), et ajoutée d'autre part à une phase lipidique comme décrit précédemment (deuxième variante).

Les résultats des mesures granulométriques avant/après lyophilisation ont permis de constater un résultat tout à fait positif pour chacune des dispersions selon l'invention testées, quelle que soit la variante de procédé utilisée pour la préparer. Par contre, le test d'une dispersion témoin constituée des vésicules seules en dispersion, obtenues à partir de la même phase lipidique, n'a conduit à aucun résultat positif (recristallisation massive du cholestérol et vésicules résiduelles de taille importante).

Par ailleurs, on a testé l'effet véhicule à travers la peau d'une dispersion selon l'invention appliquée par voie topique, pour un composé contenu dans les vésicules, dans les conditions suivantes.

On a formé une dispersion selon l'invention comprenant, par rapport au poids total de la dispersion, 5 % en poids de phase lipidique, 0,5 % en poids de polymère (soit un rapport pondéral de polymère à la phase lipidique de 1:10) et 0,3 % en poids d'un composé détectable par la technique RPE ou "sonde RPE", contenu dans les vésicules.

La phase lipidique avait pour composition : stéarate de polyéthylène glycol 400/cholestérol/"Acylglutamate HS 21" selon les proportions 45/45/10. Le polymère en dispersion était le polymère à groupes sulfoniques "AQ38" commercialisé par la Société EASTMAN CHEMICAL. La sonde RPE était l'ASL (l'iodure de N-(1-oxyl-2,2,6,6-tétraméthyl-4-pipéridinyl)-N-diméthyl-N-hydroxyéthylammonium) qui a pour formule :

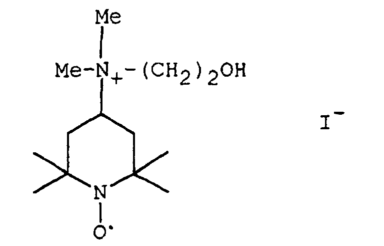

On a préparé cette dispersion selon l'invention en formant tout d'abord, de manière connue, une dispersion aqueuse de vésicules à partir de la phase lipidique et d'une solution aqueuse d'eau déminéralisée contenant la sonde RPE, cette dernière étant ainsi encapsulée dans les vésicules de la dispersion formée.

On a ensuite mélangé cette dispersion de vésicules au polymère sous forme de particules en dispersion dans de l'eau déminéralisée (première variante).

Le tableau I ci-après donne pour la dispersion selon l'invention ainsi préparée, le coefficient de diffusion D de la sonde ASL dans le stratum corneum et dans l'épiderme/derme ainsi que la pénétration ΔL 25-5.

Les mesures du coefficient de diffusion D et de la pénétration ΔL 25-5 ont été effectuées par combinaison de deux méthodes utilisant une sonde paramagnétique : la résonance paramagnétique électronique (RPE) unidimensionnelle et périodique d'une part et l'imagerie cinétique RPE d'autre part. Ces deux méthodes sont décrites respectivement dans les articles International Journal of Pharmaceutics, 62 (1990), pages 75-79, Elsevier de V. Gabrijelcic et al. "Evaluation of liposomes as drug carriers into the skin by one-dimensional EPR imaging" et Periodicum Biologorum, Vol.93, n°2, pages 245-246, (1991) de V. Gabrijelcic et al. "Liposome entrapped molecules penetration into the skin measured by nitroxide reduction kinetic imaging".

Chacune des mesures a été effectuée quatre fois.

**TABLEAU I**

| **Mesures RPE** | **Sonde ASL seule** | **Dispersion témoin de vésicules** | **Dispersion selon l'invention : Dispersion témoin + polymère "AQ38" en dispersion** |
|---|---|---|---|
| Coefficient de diffusion D : (× 10⁷; en cm²/s) stratum corneum épiderme/derme | 1,0 | 41,6 | 58 |
| | 4,2 | 33 | 33 |
| Pénétration ΔL 25-5 | 0,00 | 0,12±0,01 | 0,17±0,02 |

Il résulte du tableau I ci-dessus que la valeur du coefficient D de diffusion de la sonde ASL dans le stratum corneum dans la dispersion témoin, c'est-à-dire sans la présence du polymère en dispersion, est augmentée de plus de 39 % lorsque cette même sonde ASL est encapsulée dans les vésicules de la dispersion selon l'invention. Par ailleurs, la valeur de la pénétration ΔL 25-5 de la sonde ASL encapsulée dans la dispersion témoin est augmentée de plus de 40 % lorsque cette même sonde ASL est encapsulée dans les vésicules de la dispersion selon l'invention.

Des résultats comparables ont été obtenus avec une dispersion selon l'invention comprenant les mêmes composants mais préparée selon la deuxième variante telle que décrite précédemment (particules du polymère également présentes dans la phase aqueuse encapsulée des vésicules).

Ces résultats peuvent s'expliquer par une résistance à la déshydratation améliorée lors de l'application de la dispersion sur la peau. L'effet véhicule ainsi amélioré permet de rendre plus efficace une composition cosmétique ou pharmaceutique destinée à une application topique dont le composé cosmétiquement ou pharmaceutiquement actif est contenu dans les vésicules.

La dispersion selon l'invention peut ainsi contenir au moins un composé cosmétiquement ou pharmaceutiquement actif, soit dans la phase aqueuse encapsulée des vésicules si ledit actif est hydrosoluble, soit dans la phase lipidique des vésicules s'il est oléosoluble. Si l'actif est amphiphile, il se répartit alors entre la phase lipidique et la phase aqueuse encapsulée de chaque vésicule, selon son coefficient de partage. Selon l'invention, un composé cosmétiquement ou pharmaceutiquement actif peut également être présent dans le milieu aqueux de la dispersion.

Les divers actifs pouvant être présents dans les vésicules ou dans le milieu aqueux de la dispersion selon l'invention, qu'ils soient hydrophiles, lipophiles ou amphiphiles, sont notamment ceux décrits dans le tableau I de FR-A-2694884.

Le milieu aqueux de la dispersion selon l'invention peut en outre contenir des additifs conventionnels utilisés pour la formulation de la dispersion sous forme de lotion, crème ou sérum. Ces adjuvants sont, en particulier, pris dans le groupe formé par les gélifiants, les conservateurs, les colorants, les opacifiants et les parfums. Parmi les gélifiants utilisables, on peut citer les dérivés de cellulose tels que l'hydroxyéthylcellulose, les dérivés d'algues tels que le satiagum, des gommes naturelles, telles que l'adragante, et des polymères synthétiques, en particulier les mélanges d'acides polycarboxyvinyliques commercialisés sous la dénomination "Carbopol" par la Société GOODRICH et le mélange de copolymères acrylate de Na/acrylamide commercialisé sous la dénomination "Hostacerin PN73" par la Société HOECHST.

La dispersion selon l'invention peut aussi être utilisée pour disperser des huiles. Comme huiles, on peut citer par exemple les huiles d'origine végétale, les huiles minérales, les huiles synthétiques et les huiles siliconées.

La présente invention a donc également comme objet une composition cosmétique ou pharmaceutique à application topique, caractérisée par le fait qu'elle est essentiellement constituée par une dispersion selon l'invention, dont les vésicules contiennent au moins un composé cosmétiquement ou pharmaceutiquement actif.

Par application topique, on entend selon l'invention une application locale de la composition sur la peau, les phanères, les muqueuses ou les cheveux.

Les compositions cosmétiques ou pharmaceutiques selon l'invention se sont avérées présenter un intérêt tout particulier par application sur la peau, y compris le cuir chevelu. Elles favorisent le soin de la peau et notamment l'hydratation, la nutrition, la protection et le raffermissement. Selon le ou les actifs utilisés, elles ont par ailleurs un effet anti-âge, anti-rides, amincissant, dépigmentant, anti-acné et favorisent les traitements des mycoses, des dermites et du psoriasis. Elles peuvent aussi être utilisées comme compositions de maquillage de la peau.

La présente invention a donc également pour objet l'utilisation cosmétique d'une composition telle que définie ci-dessus pour le soin et/ou le maquillage de la peau ou pour le soin du cuir chevelu et/ou des cheveux.

La présente invention a en outre pour objet un procédé de traitement non-thérapeutique de la peau ou du cuir chevelu, caractérisé par le fait qu'il consiste à appliquer sur la peau ou sur le cuir chevelu une composition telle que définie ci-dessus.

On va maintenant donner à titre d'illustration des exemples de préparation d'une dispersion selon l'invention et de compositions obtenues à partir d'une telle dispersion.

Dans ce qui suit, les proportions des divers composants sont exprimées en pourcentage pondéral, par rapport au poids total de la dispersion finale selon l'invention.

### EXEMPLE 1 :Préparation d'une dispersion selon l'invention

Une phase lipidique constituée des composants suivants :
- Stéarate de polyéthylène glycol 400
   (commercialisé par la Société UNICHEMA) 1,9 %
- Cholestérol 1,9 %
- "Acylglutamate HS 11" (sel monosodique de l'acide N-stéaroylglutamique commercialisé par la Société AJINOMOTO) 0,2 %
est portée à une température de 110°C sous agitation afin d'effectuer une pré-association par cofusion des lipides puis le mélange ainsi obtenu est ramené à température ambiante.

La phase lipidique ainsi obtenue est alors introduite (deuxième variante), sous agitation rapide (barreau magnétique/Moritz), dans une dispersion de polymère sous forme de particules, maintenue à une température de 80°C, et ayant la composition suivante :
- Polyester à groupes sulfoniques "AQ 38" (commercialisé par la Société Eastman Chemical ; taille moyenne des particules comprise entre 5 et 60 nm) 0,4 %
- Eau déminéralisée q.s.p. 100 %

On laisse agiter pendant une heure. On fait ensuite passer la dispersion à deux reprises dans un homogénéisateur haute pression à 500 bars, à température ambiante. La taille moyenne des vésicules est finalement de l'ordre de 200 nm.

La dispersion selon l'invention ainsi obtenue est soumise à une opération de lyophilisation pour tester de manière extrême la résistance à la déshydratation des vésicules, comme décrit ci-dessus.

Après réhydratation avec de l'eau déminéralisée, on constate d'une part, qu'il n'y a pas de recristallisation de lipide et que la dispersion est fine et homogène et, d'autre part, que la taille moyenne des vésicules est inférieure au double de la taille moyenne initiale des vésicules : le test de lyophilisation s'avère donc positif.

On obtient le même résultat au test de lyophilisation en mélangeant le polymère en dispersion tel que décrit ci-dessus à une dispersion aqueuse de vésicules déjà formée à partir d'eau déminéralisée et de la phase lipidique également décrite ci-dessus (première variante), en utilisant les mêmes proportions de chaque composant par rapport au poids total de la dispersion finale.

Des dispersions du même type que celles obtenues ci-dessus ont été réalisées selon un mode opératoire identique mais en utilisant d'autres substances pour former la phase lipidique et divers types de polymères pour former la dispersion. Parmi ces derniers on peut notamment mentionner les polyesters à groupes sulfoniques tels que ceux commercialisés sous les dénominations "AQ 29", "AQ 1350", "AQ 1950" et "AQ 14000" par la société Eastman Chemical ainsi que ceux commercialisés sous les dénominations de "Polycare" par la société Rhône Poulenc.

### EXEMPLE 2 : Composition de sérum anti-âge pour le visage

On réalise une dispersion selon l'invention comme décrit à l'exemple 1 en incorporant, lors de la formation des vésicules, les principes actifs classiques d'un sérum anti-âge, pour obtenir la composition suivante :

### Phase lipidique :

- Stéarate de polyéthylène glycol 400 1,425 %
- Cholestérol 1,425 %
- "Acylglutamate HS 11" 0,15 %

### Polymère en dispersion :

- Polyester à groupes sulfoniques "AQ38" 0,3 %
- Glycérol 3 %
- L-hydroxyproline 1 %
- D-panthénol 1,5 %
- Guanosine 0,01 %
- L-lysine monohydrate q.s.p. pH = 6,5
- Eau déminéralisée q.s.p. 100 g

Après formation de la dispersion, on y ajoute 0,3 % d'un agent gélifiant (mélange d'acides polycarboxyvinyliques : "Carbopol 940" commercialisé par la Société GOODRICH).

Dans cet exemple les principes actifs de la composition peuvent être avantageusement remplacés par d'autres principes actifs pour les soins ou le traitement de la peau tels que par exemple des actifs anti-rides des anti-acnéiques ou des substances à action amincissante.

## Revendications

1. Dispersion, dans un milieu aqueux, de vésicules résistantes à la déshydratation constituées d'une phase lipidique et d'une phase aqueuse encapsulée, **caractérisée par le fait qu'**elle contient en outre, soit dans ledit milieu aqueux, soit dans ladite phase aqueuse encapsulée ou dans les deux en même temps, au moins un polymère en dispersion sous forme de particules, ledit polymère étant choisi dans le groupe constitué par les polymères ayant une température de transition vitreuse Tv, en présence ou non d'un agent plastifiant, inférieure à 70°C à 0 % % d'humidité relative ou inférieure à 55°C à 65 % d'humidité relative.

2. Dispersion selon la revendication 1, **caractérisée par le fait que** ledit polymère est choisi parmi les polymères synthétiques, les polymères d'origine naturelle et leurs mélanges.

3. Dispersion selon la revendication 1 ou 2, **caractérisée par le fait que** ledit polymère est choisi dans le groupe constitué par les polyesters, les polyuréthannes, les polymères à fonctions acides carboxyliques, les polymères vinyliques, la résine shellac, la gomme de sandarac, les dammars, les élémies, les copals, les dérivés cellulosiques, et leurs mélanges.

4. Dispersion selon l'une quelconque des revendications précédentes, **caractérisée par le fait que** ledit polymère est choisi dans le groupe constitué par les polyesters à groupes sulfoniques, les polyesters à groupes sulfoniques contenant en outre des motifs dérivés d'éthylène glycol, de tri- et tétra-éthylène glycol et de téréphtalate, les polyesters à fonction amine terminale neutralisés, les copolymères séquencés polyesters du type acide phtalique et sulfophtalique/éthylène glycol/polyméthyl siloxane α,γ-hydroxypropyl et les copolymères acétate de vinyle/butyl-benzoate de vinyle/acide crotonique.

5. Dispersion selon l'une quelconque des revendications précédentes, **caractérisée par le fait que** ledit polymère comprend un agent plastifiant.

6. Dispersion selon la revendication 5, **caractérisée par le fait que** ledit agent plastifiant est choisi dans le groupe constitué par le dipropylène glycol méthyléther, le tripropylène glycol méthyléther, l'adipate de diéthyle, l'adipate de diisopropyle et le triacétate de glycérol.

7. Dispersion selon l'une quelconque des revendications précédentes, **caractérisée par le fait que** ledit polymère en dispersion est tel que la taille moyenne des particules dudit polymère est comprise entre 5 et 2000 nm.

8. Dispersion selon l'une quelconque des revendications précédentes, **caractérisée par le fait que** la taille moyenne des vésicules est comprise entre 10 et 5000 nm.

9. Dispersion selon l'une quelconque des revendications précédentes, **caractérisée par le fait que** la concentration dudit polymère en dispersion est comprise entre 0,01 et 30 % dudit polymère par rapport au poids total de la dispersion.

10. Dispersion selon l'une quelconque des revendications précédentes, **caractérisée par le fait que** la concentration des vésicules est comprise entre 0,1 et 30 % en poids de ladite phase lipidique par rapport au poids total de la dispersion.

11. Dispersion selon l'une quelconque des revendications précédentes, **caractérisée par le fait que** le rapport pondéral dudit polymère à ladite phase lipidique est compris entre 1:500 et 20:1.

12. Dispersion selon la revendication 11, **caractérisée par le fait que** ledit rapport pondéral est compris entre 1:100 et 1:1.

13. Composition cosmétique ou pharmaceutique à application topique, **caractérisée par le fait qu'**elle est essentiellement constituée par une dispersion selon l'une quelconque des revendications 1 à 12, dont les vésicules contiennent au moins un composé cosmétiquement ou pharmaceutiquement actif.

14. Utilisation cosmétique de la composition cosmétique selon la revendication 13 pour le soin et/ou le maquillage de la peau ou pour le soin du cuir chevelu et/ou des cheveux.

15. Procédé de traitement non-thérapeutique de la peau ou du cuir chevelu, **caractérisé par le fait qu'**il consiste à appliquer sur la peau ou sur le cuir chevelu une composition cosmétique selon la revendication 13.

## Claims

1. Dispersion, in an aqueous medium, of dehydration-resistant vesicles consisting of a lipid phase and of an encapsulated aqueous phase, **characterized in that** it additionally contains, either in the said aqueous medium or in the said encapsulated aqueous phase, or in both at the same time, at least one polymer in dispersion in the form of particles, the said polymer being chosen from the group consisting of polymers which have a glass transition temperature Tg, in the presence or absence of a plasticizing agent, lower than 70°C at 0% relative humidity or lower than 55°C at 65% relative humidity.

2. Dispersion according to Claim 1, **characterized in that** the said polymer is chosen from synthetic polymers, polymers of natural origin and mixtures thereof.

3. Dispersion according to Claim 1 or 2, **characterized in that** the said polymer is chosen from the group consisting of polyesters, polyurethanes, polymers containing carboxylic acid functional groups, vinyl polymers, shellac resin, sandarac gum, dammars, elemis, copals, cellulose derivatives and mixtures thereof.

4. Dispersion according to any one of the preceding claims, **characterized in that** the said polymer is chosen from the group consisting of polyesters containing sulphonic groups, polyesters containing sulphonic groups additionally containing units derived from ethylene glycol, from tri- and tetraethylene glycol and from terephthalate, neutralized polyesters containing a terminal amine functional group, polyester block copolymers of the phthalic and sulphophthalic acid/ethylene glycol/polymethyl siloxane α,γ-hydroxy-propyl type and vinyl acetate/vinyl butylbenzoate/crotonic acid copolymers.

5. Dispersion according to any one of the preceding claims, **characterized in that** the said polymer includes a plasticizing agent.

6. Dispersion according to Claim 5, **characterized in that** the said plasticizing agent is chosen from the group consisting of dipropylene glycol methyl ether, tripropylene glycol methyl ether, diethyl adipate, diisopropyl adipate and glycerol triacetate.

7. Dispersion according to any one of the preceding claims, **characterized in that** the said polymer in dispersion is such that the mean size of the particles of the said polymer is between 5 and 2000 nm.

8. Dispersion according to any one of the preceding claims, **characterized in that** the mean size of the vesicles is between 10 and 5000 nm.

9. Dispersion according to any one of the preceding claims, **characterized in that** the concentration of the said polymer in dispersion is between 0.01 and 30 % of the said polymer relative to the total weight of the dispersion.

10. Dispersion according to any one of the preceding claims, **characterized in that** the vesicle concentration is between 0.1 and 30 % by weight of the said lipid phase relative to the total weight of the dispersion.

11. Dispersion according to any one of the preceding claims, **characterized in that** the weight ratio of the said polymer to the said lipid phase is between 1:500 and 20:1.

12. Dispersion according to Claim 11, **characterized in that** the said weight ratio is between 1:100 and 1:1.

13. Cosmetic or pharmaceutical composition for topical application, **characterized in that** it consists essentially of a dispersion according to any one of Claims 1 to 12, in which the vesicles contain at least one cosmetically or pharmaceutically active compound.

14. Cosmetic use of the cosmetic composition according to Claim 13 for the care and/or make-up of the skin or for the care of the scalp and/or hair.

15. Process for nontherapeutic treatment of the skin or of the scalp, **characterized in that** it consists in applying to the skin or to the scalp a cosmetic composition according to Claim 13.

## Patentansprüche

1. Dispersion in einem wässrigen Milieu von gegen Dehydrierung beständigen Vesikeln, bestehend aus einer Lipidphase und einer verkapselten wässrigen Phase, **dadurch gekennzeichnet, dass** diese zusätzlich sei es im wässrigen Milieu sei es in der entkapselten wässrigen Phase oder in beiden derselben gleichzeitig mindestens ein Polymer in Dispersion in Form von Teilchen enthält, wobei das Polymer ausgewählt ist aus der Gruppe, bestehend aus Polymeren mit einer Glasübergangstemperatur Tv, in Anwesenheit eines Weichmachers oder nicht, unterhalb von 70 °C bei 0 % relativer Feuchtigkeit oder unterhalb 55 °C bei 65 % relativer Feuchtigkeit.

2. Dispersion gemäß Anspruch 1, **dadurch gekennzeichnet, dass** das Polymer ausgewählt ist unter den synthetischen Polymeren, den Polymeren natürlichen Ursprungs oder deren Mischungen.

3. Dispersion gemäß Anspruch 1 oder 2, **dadurch gekennzeichnet, dass** das Polymer ausgewählt ist aus der Gruppe, bestehend aus den Polyestern, den Polyurethanen, den Polymeren mit Carbonsäurefunktionen, Vinylpolymeren, dem Shellackharz, Sandarakgummi, den Dammarharzen, den Elemieharzen, den Copalharzen, cellulosischen Derivaten und deren Mischungen.

4. Dispersion gemäß einem der vorstehenden Ansprüche, **dadurch gekennzeichnet, dass** das Polymer ausgewählt ist aus der Gruppe, bestehend aus den Polyestern mit Sulfonsäuregruppen, den Polyestern mit Sulfonsäuregruppen, enthaltend zusätzlich Einheiten, abgeleitet von Ethylenglycol, von Tri- und Tetraethylenglycol und von Terephthalat, den Polyestern mit neutralisierten endständigen Aminfunktionen, den Blockcopolymeren von vom Typ der Phthalsäure- oder Sulfophthalsäure-Polyester/Ethylenglycol/ α,γ-Hydroxypropylpolymethylsiloxan und den Copolymeren von Vinylacetat/Vinylbutylbenzoat/Crotonsäure.

5. Dispersion gemäß einem der vorstehenden Ansprüche, **dadurch gekennzeichnet, dass** das Polymer einen Weichmacher enthält.

6. Dispersion gemäß Anspruch 5, **dadurch gekennzeichnet, dass** der Weichmacher ausgewählt ist aus der Gruppe, bestehend aus Dipropylenglycolmethylether, Tripropylenglycolmethylether, Diethyladipat, Diisopropyladipat und Glyceroltriacetat.

7. Dispersion gemäß einem der vorstehenden Ansprüche, **dadurch gekennzeichnet, dass** das Polymer so dispegiert ist, dass der mittlere Durchmesser der Teilchen des Polymeren zwischen 5 und 2000 nm liegt.

8. Dispersion gemäß einem der vorstehenden Ansprüche, **dadurch gekennzeichnet, dass** der mittlere Durchmesser der Vesikel zwischen 10 und 5000 nm liegt.

9. Dispersion gemäß einem der vorstehenden Ansprüche, **dadurch gekennzeichnet, dass** die Konzentration des Polymeren in Dispersion zwischen 0,01 und 30 % des Polymeren liegt, bezogen auf das Gesamtgewicht der Dispersion.

10. Dispersion gemäß einem der vorstehenden Ansprüche, **dadurch gekennzeichnet, dass** die Konzentration der Vesikel zwischen 0,1 und 30 Gew.-% der Lipidphase, bezogen auf das Gesamtgewicht der Dispersion, liegt.

11. Dispersion gemäß einem der vorstehenden Ansprüche, **dadurch gekennzeichnet, dass** das Gewichtsverhältnis des Polymeren zur Lipidphase zwischen 1:500 und 20:1 liegt.

12. Dispersion gemäß Anspruch 11, **dadurch gekennzeichnet, dass** das Gewichtsverhältnis zwischen 1:100 und 1:1 liegt.

13. Kosmetische oder pharmazeutische Zubereitung zur topischen Anwendung, **dadurch gekennzeichnet, dass** sie im Wesentlichen aus einer Dispersion gemäß einem der Ansprüche 1 bis 12 besteht, wobei die Vesikel mindestens eine kosmetisch oder pharmazeutisch aktive Verbindung enthalten.

14. Kosmetische Verwendung der kosmetischen Zubereitung gemäß Anspruch 13 für die Pflege und/oder das Schminken der Haut oder für die Pflege der Kopfhaut und/oder der Haare.

15. Verfahren zur nicht-therapeutischen Behandlung der Haut oder der Kopfhaut, **dadurch gekennzeichnet, dass** es aus dem Anwenden einer kosmetischen Zubereitung gemäß Anspruch 13 auf die Haut oder auf die Kopfhaut besteht.
